# EUROPEAN PATENT APPLICATION

(11) **EP 4 215 186 A1**
(43) Date of publication of application: **26.07.2023**
(21) Application number: 22212490.1
(22) Date of filing: 08.02.2019
(51) Int. Cl.: A61K 9/14, A61K 9/16, A61K 9/127, A61K 39/35, A61K 39/385, A61P 37/02

(54) **TREATMENT OF CELIAC DISEASE WITH TOLERIZING PARTICLES**

(30) Priority: 08.02.2018 US 201862628233 P
(62) Divisional of application: 19751688.3
(71) Applicant: Cour Pharmaceuticals Development Company Inc., Northbrook, Illinois 60062 (US)
(72) Inventor: GETTS, Daniel R., Northbrook 60062 (US)
(74) Representative: D Young & Co LLP

(57) **Abstract**

The present disclosure relates to methods for treating Celiac Disease using tolerizing immune modifying particles that encapsulate antigenic material from the gliadin protein or other related proteins.

## Description

### CROSS REFERENCE TO RELATED APPLICATIONS

This application claims the priority benefit of U.S. Provisional Patent Application No. 62/628,233, filed February 8, 2018, hereby incorporated by reference in its entirety.

### FIELD OF THE DISCLOSURE

The present disclosure relates to methods for treating Celiac disease using tolerizing immune modifying particles that encapsulate antigenic material from the gliadin protein or other related proteins.

### INCORPORATION BY REFERENCE OF MATERIAL SUBMITTED ELECTRONICALLY

This application contains, as a separate part of disclosure, a sequence listing in computer-readable form (filename: 52341_Seqlisting.txt; 6,713 bytes; created February 8, 2019) which is incorporated by reference in its entirety.

### BACKGROUND

Celiac disease (CD) is a common immunological disorder with an estimated prevalence of 0.3 to 2.4% among people of European ancestry (Fasano 2012). It develops in genetically predisposed subjects as a consequence of an abnormal T cell response to dietary prolamin protein, predominately gliadin, which becomes deamidated by tissue transglutaminase in the intestine. When deamidated gliadin-specific CD4+ T cells recognize their cognate gliadin epitope presented by human leukocyte antigen (HLA)-DQ/8 or HLA-DQ2 on antigen-presenting cells (APCs) in the lamina propria, they become activated and produce proinflammatory cytokines such as interferon-gamma (IFN-γ). This triggers an inflammatory cascade resulting in crypt hyperplasia and villous flattening characteristic of CD biopsy findings. Within the intestinal epithelium gliadin also triggers local production of interleukin-15 (IL-15) by enterocytes. This IL-15 increases expression of major histocompatibility complex (MHC) class I surface antigens (such as MHC class I polypeptide-related sequence A) on epithelial cells and also increases expression of corresponding MHC receptors (such as NKG2D) on intraepithelial T cells (i.e., CD8+αβ T cells and γδ T cells, natural killer [NK] cells), leading to epithelial cell destruction. (Fasano 2012; Green 2007; Leffler 2017; Mazzarela 2008; Tack 2010)

The enteropathy resulting from the T cell activation produces the chronic diarrhea, abdominal distension/pain, constipation, and other gastrointestinal symptoms, increased intestinal permeability, malabsorption, and occult gastrointestinal bleeding commonly observed in CD. Other manifestations might include weight loss, chronic fatigue, osteoporosis, refractory iron deficiency, anemia, infertility, growth failure in children, arthritis, peripheral neuropathy, dermatitis herpetiformis, gluten ataxia, and malignancy (Farrell 2002; Fasano 2012; Fasano 2001; Green 2007; Leffler 2017). In rare cases CD patients, primarily children, experience a life-threatening metabolic emergency termed celiac crisis, characterized by hypokalemia and acidosis secondary to profuse diarrhea (Baranwal 2003, Farrell 2002; Fasano 2012).

Gluten avoidance by dietary modification, the "gluten free" diet (GFD), is the only effective treatment for CD as there are currently no medications that can reliably and safely prevent the mucosal damage caused by exposure to gluten. While the GFD has been shown to alleviate many of the symptoms of disease, strict adherence is difficult, and creates an additional burden on the day-to-day functioning of the celiac patient. In reality, the complete elimination of gluten from the diet is not realistic and repeated gluten exposure, albeit unintentional or in small amounts, prevents complete recovery of symptoms and repair of intestinal damage (Laurin 2002; Leffler 2017; Rubio-Tapia 2013).

International Patent Publication WO 2010/060155 discloses different epitopes of wheat proteins that may be involved in Celiac Disease and describes that all gluten proteins are considered toxic in celiac disease. In 2006, the NCBI public database Genbank included 345 entries for gluten proteins from bread-making wheat (*Triticum aestivum*)*,* barley (*Hordein vulgare*) and rye (*Secale cerale*)*.*

Numerous therapies have been designed to induce immune tolerance in autoimmune diseases. However, these treatments have yielded only marginal efficacy in clinical trials (Kaukinen 2014).

### SUMMARY

Therapeutic approaches rendering T cells tolerant to gluten could potentially cure CD, thus eliminating the burdens associated with lifetime GFD and co-morbidities associated with disease such as cancer.

Provided herein is a method of treating Celiac Disease in a subject comprising administering to the subject a tolerizing immune modifying particle (TIMP) encapsulating one or more gliadin antigenic epitopes (GLIA), wherein the particle is administered at a dose of 0.1 to 10 mg/kg.

Also provided herein is a method for reducing sensitivity to gluten in a subject comprising administering to the subject a tolerizing immune modifying particle (TIMP) encapsulating one or more gliadin antigenic epitopes (GLIA), wherein the particle is administered at a dose from about 0.1 to about10 mg/kg. In various embodiments, the TIMP-GLIA is administered at a dose of about 0.1 mg/kg, 0.5 mg/kg, 1.0 mg/kg, 2.0 mg/kg, 4.0 mg/kg, 8.0 mg/kg or 10 mg/kg. In various embodiments, the TIMP-GLIA is administered at a dose of about 8.0 mg, 80 mg, 320 mg, 640 mg or 800 mg.

In various embodiments, the TIMP-GLIA is administered in a single dose or in multiple doses.

In various embodiments, the TIMP-GLIA is administered intravenously, subcutaneously, intramuscularly, intraperitoneally or orally.

In various embodiments, the TIMP-GLIA comprises Poly(lactic-co-glycolic acid) (PLGA). In various embodiments, the TIMP-GLIA is carboxy functionalized on the surface, i.e., carboxylated PLGA.

In various embodiments, the TIMP-GLIA has a negative zeta potential. In various embodiments, the zeta potential is between -80 to -30 mV, or between -70 mV and -30 mV , or between -60 mV and -35 mV, or between -50 to -40 mV.

In various embodiments, the TIMP-GLIA comprises Poly(lactic-co-glycolic acid) (PLGA) with a copolymer ratio of about 50:50 of polylactic acid:polyglycolic acid. Additional contemplated copolymer ratios are described in the Detailed description.

In various embodiments, the particle size is between 100 and 1500 nm, or between 100 and 1000 nm, or between 300 to 1000 nm, or between 400 and 800 nm, or between 200 and 700 nm.

In various embodiments, the one or more GLIA antigens are selected from the group consisting of gliadin, glutenin, hordein, and secalin or antigenic fragments or epitopes thereof. Exemplary antigenic epitopes that can induce gluten sensitivity and Celiac disease contemplated for use in the particle are described in greater detail in the Detailed Description.

In various embodiments, the subject is on a gluten free diet. In various embodiments, the subject has a genetic profile of HLA-DQ2.5 (HLA-DQA1*0501/B1*0201) or HLA-DQ8.1 (DQA1*0301/Br0302). In various embodiments, the subject has Refractory Celiac Disease.

In various embodiments, the administration of the TIMP-GLIA particle improves one or more signs or symptoms of Celiac Disease or gluten sensitivity. Exemplary symptoms, include , but are not limited to the one or more symptoms is selected from the group consisting of weight loss, fatigue, headache, iron deficiency, folic acid deficiency, vitamin D deficiency, vitamin B12 deficiency, villous atrophy, intestinal mucosal damage, and low bone density. One or more symptoms include subjective symptoms such as villous atrophy, tetramer staining, ELISPOT, miRNA, Exosomes, RNA, and DNA.In various embodiments, the TIMP-GLIA is infused over 30 minutes, 1 hour, 2 hours, 3 hours or more.

In various embodiments, the TIMP-GLIA is infused at escalating rates. In various embodiments, the escalating rate doubles after 15 minutes of initial infusion. In various embodiments, the escalating rate doubles after the second 15 minutes of infusion, e.g., is 4 times the initial infusion rate. In some embodiments, the TIMP-GLIA is infused over approximately 2.5 hours at approximately 20mL/hour for the first 15 minutes, 40 mL/hour for the next 15 minutes, and 80 mL/hour for the duration of the infusion.

In various embodiments, dose escalating administration of TIMP-GLIA reduces complement activation compared to non escalating dose administration.

In various embodiments, the TIMP-GLIA is administered once weekly, once every two weeks, once every three weeks, once every 4 weeks, once every two months, once every three months, once every 6 months or once per year.

In various embodiments, the TIMP-GLIA further comprises a pharmaceutical acceptable carrier, diluent or excipient.

In various embodiments, the administration result in apoptosis of macrophages or monocytes in the subject. In various embodiments, the administration induces immunologic anergy.

In various embodiments, the TIMP-GLIA is administered in conjunction with a second agent.

In various embodiments, the TIMP-GLIA is administered as a booster dose. In various embodiments, the booster dose is administered when needed as determined by an analytical test, e.g., by skin-prick test or measurement of peripheral blood mononuclear cell levels.

### BRIEF DESCRIPTION OF THE DRAWING

Figure 1 is a schematic showing the dose schedule for Celiac patients.
Figure 2 is a schematic showing the dose schedule for healthy subjects.
Figure 3 is a schematic showing an alternate dose schedule for Celiac patients or healthy volunteers.

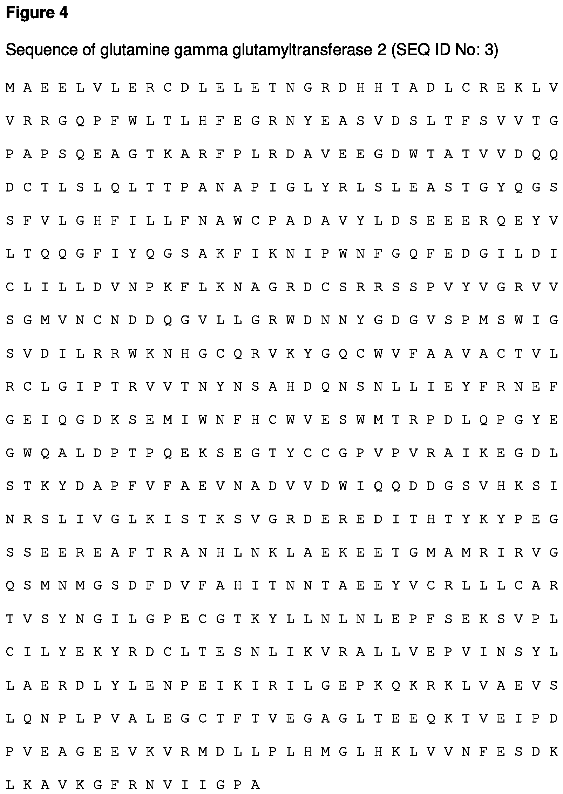

Figure 4 shows the sequence of the glutamine gamma glutamyltransferase 2 protein (SEQ ID NO: 3).
Figures 5A-5B show updated schematics for administration of TIMP-GLIA to subjects in single (Figure 5A) and repeat dosing (Figure 5B) groups.
Figures 6A-6F show plasma TIMP-GLIA concentrations over time for Part A individual subjects, plotted by dose.

### DETAILED DESCRIPTION

The present disclosure provides a dosing regimen for the treatment of Celiac Disease using tolerizing immune modifying particles that encapsulate antigenic epitopes relevant to Celiac Disease and sensitivity to gluten.

### Definitions

"Particle" as used herein refers to any non-tissue derived composition of matter, it may be a sphere or sphere-like entity, bead, or liposome. The term "particle", the term "immune modifying particle", the term "carrier particle", and the term "bead" may be used interchangeably depending on the context. Additionally, the term "particle" may be used to encompass beads and spheres.

"TIMP" as used herein refers to tolerizing immune modifying particles which are coupled to an antigen. In some embodiments, the antigen is attached to the surface of the TIMP. In other embodiments, the antigen is encapsulated within the TIMP.

"Negatively charged particle" as used herein refers to particles which have been modified to possess a net surface charge that is less than zero.

"Carboxylated particles" or "carboxylated beads" or "carboxylated spheres" includes any particle that has been modified to contain a carboxyl group on its surface. In some embodiments the addition of the carboxyl group enhances phagocyte/monocyte uptake of the particles from circulation, for instance through the interaction with scavenger receptors such as MARCO. Carboxylation of the particles can be achieved using any compound which adds carboxyl groups, including, but not limited to, Poly(ethylene-maleic anhydride) (PEMA).

"Antigenic moiety" as used herein refers to any moiety, for example a peptide, that is recognized by the host's immune system. Examples of antigenic moieties include, but are not limited to, gliadin , gliadin epitopes, and other related proteins as disclosed herein.

The term "epitope" refers to that portion of any molecule capable of being recognized by and bound by a selective binding agent at one or more of the antigen binding regions and may cause an immune reaction. Epitopes usually consist of chemically active surface groupings of molecules, such as, amino acids or carbohydrate side chains, and have specific three-dimensional structural characteristics as well as specific charge characteristics. Epitopes as used herein may be contiguous or non-contiguous/discontinuous. Exemplary discontinuous epitopes for gliadin are described in Table 3 of US Patent 9,616,113.

"Peptides" or "oligopeptides" are short amino acid sequences, typically between 3 and 100 amino acid residues in length and encompass naturally occurring amino acid residues and non-naturally occurring analogs of residues which may be used singly or in combination with naturally occurring amino acid residues in order to give the peptide a particular conformational specificity or a particular biological activity, such as resistance to proteolysis. Peptides include repeats of peptide sequences and may include 2, 3, 4, 5, 6, 7, 8, 9, 10 or more copies of an amino acid sequence arranged head-to-tail or head-to-head. Peptides include dimers, trimers or higher order multimers, e.g. formed through conjugation to other polymeric or non-polymeric moieties, such as PEG.

"Polypeptides" are longer amino acid sequences, typically 100 or more amino acid residues in length, and encompass naturally occurring amino acid residues and non-naturally occurring analogs of residues which may be used singly or in combination with naturally occurring amino acid residues in order to give the polypeptide a particular conformational specificity or a particular biological activity, such as resistance to proteolysis.

"Naked beads" or "naked particles" or "naked spheres" as used herein refers to beads, particles or spheres that have not been carboxylated.

"Pro-inflammatory mediators" or "pro-inflammatory polypeptides" as used herein refers to polypeptides or fragments thereof which induce, maintain, or prolong inflammation in a subject. Examples of pro-inflammatory mediators include, but are not limited to, cytokines and chemokines.

The term "Inflammatory monocyte" as used herein refers to any myeloid cell expressing any combination of CD14/CD26 and CCR2.

The term "inhibitory neutrophil" as used herein refers to neutrophils, and/or monocyte derived suppressor cells.

The term "Th cell" or "helper T cell" as used herein refers to CD4⁺ cells. CD4⁺ T cells assist other white blood cells with immunologic processes, including maturation of B cells into plasma cells and memory B cells, and activation of cytotoxic T cells and macrophages. T cells become activated when they are presented with peptide antigens by MHC class II molecules, which are expressed on the surface of antigen-presenting cells (APCs).

As used herein, the term "Th1 cell" refers to a subset of Th cells which produce proinflammatory mediators. Th1 cells secrete cytokines to facilitate immune response and play a role in host defense against pathogens in part by mediating the recruitment of neutrophils and macrophages to infected tissues. Th1 cells secrete cytokines including IFN-gamma, IL2, IL-10, and TNF alpha/beta to coordinate defense against intracellular pathogens such as viruses and some bacteria.

As used herein, the term "Th2 cell" refers to a subset of Th cells that mediate the activation and maintenance of the antibody-mediated immune response against extracellular parasites, bacteria, allergens, and toxins. Th2 cells mediate these functions by producing various cytokines such as IL-4, IL-5, IL-6, IL-9, IL-13, and IL-17E (IL-25) that are responsible for antibody production, eosinophil activation, and inhibition of several macrophage functions, thus providing phagocyte-independent protective responses.

As used herein, the term "Th17 cell" refers to a subset of Th cells. Th17 cells secrete cytokines to facilitate immune response and play a role in host defense against pathogens by mediating the recruitment of neutrophils and macrophages to infected tissues. TH17 cells secrete cytokines such as IL-17, IL-21, IL-22, IL-24, IL-26 and TNF alpha to coordinate defense against extracellular pathogens including fungi and bacteria.

The term "therapeutically effective amount" is used herein to indicate the amount of target-specific composition of the disclosure that is effective to ameliorate or lessen symptoms or signs of the disease being treated.

The term "celiac disease" refers to a chronic inflammatory disease of the small intestine. The disease encompasses a spectrum of conditions characterized by varying degrees of gluten sensitivity, including a severe form characterized by a flat small intestinal mucosa (hyperplastic villous atrophy) and other forms characterized by milder symptoms including fatigue, chronic diarrhea, malabsorption of nutrients, weight loss, abdominal distension, anemia as well as a substantially enhanced risk for the development of osteoporosis and intestinal malignancies (lymphoma and carcinoma).

The term "sensitive to gluten" or "gluten sensitive" refers to the state in which any one or more of the symptoms of celiac disease or an inappropriate T cell response are exhibited by a subject exposed to gluten, or peptide fragment thereof. In a subject who is not sensitive to gluten, there is little or no T cell response caused by ingestion of gluten. By contrast, in a subject sensitive to gluten there is an inappropriate CD4+ T cell mediated immune response to peptides derived from gluten after ingestion thereof.

### Tolerizing Immune Modifying Particles

In some embodiments, the present disclosure provides for use of compositions comprising: an antigen coupled to or encapsulated in a carrier particle with a negative zeta potential. In some embodiments, the zeta potential of the particle is from about -100 mV to about 0 mV. In some embodiments, the zeta potential of the particle is from about -80 mV to about -30 mV, from about -70 mV to about -30 mV, from about -60 mV to about -35 mV, or from about -50 mV to about -40 mV. In some embodiments, the particle is a co-polymer having a molar ratio from about 50:50, 80:20 to about 100:0 polylactic acid:polyglycolic acid. In some embodiments, the particle is a poly(lactic-co-glycolic acid) particle.

In some embodiments, the particle has an average diameter of between about 0.1 µm to about 10 µm. In some embodiments, the particle has an average diameter of between 0.2 µm and about 2 µm. In some embodiments, the particle has a diameter of between about 0.3 µm to about 5 µm. In some embodiments, the particle has a diameter of between about 0.5 µm to about 3 µm. In some embodiments, the particle has a diameter of between about 0.5 µm to about 1 µm. In some embodiments, the particle has a diameter of about 100 to 1500 nm, abut 100 to 10000 nm, about 300 to 1000 nm, about 400 to 800 nm or about 200 to 700 nm.

Methods of making a TIMP useful in the methods of the disclosure are described in WO 2017/112899 and WO 2017/143346, incorporated herein by reference.

To administer particles as described herein to human or test mammals, it is preferable to formulate the particle in a sterile composition comprising one or more sterile pharmaceutically acceptable carriers. The phrase "pharmaceutically or pharmacologically acceptable" refer to molecular entities and compositions that do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like.

The particle is administered by any suitable means, including parenteral, subcutaneous, intraperitoneal, intrapulmonary, and intranasal. Parenteral infusions include intravenous, intraarterial, intraperitoneal, intramuscular, intradermal or subcutaneous administration. Preferably the dosing is given by injections, most preferably intravenous or subcutaneous injections, depending in part on whether the administration is brief or chronic. Other administration methods are contemplated, including topical, particularly transdermal, transmucosal, rectal, oral or local administration e.g. through a catheter placed close to the desired site.

Pharmaceutical compositions of the present disclosure containing a particle herein as an active ingredient may contain sterile pharmaceutically acceptable carriers or additives depending on the route of administration. Examples of such carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, glycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof, as appropriate, depending on the dosage form of the present invention. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. Intravenous vehicles can include various additives, preservatives, or fluid, nutrient or electrolyte replenishers. A variety of aqueous carriers are suitable, e.g., sterile phosphate buffered saline solutions, bacteriostatic water, water, buffered water, 0.4% saline, 0.3% glycine, and the like, and may include other proteins for enhanced stability, such as albumin, lipoprotein, globulin, etc., subjected to mild chemical modifications or the like.

It is contemplated that the particle may further comprise a surfactant. The surfactant can be anionic, cationic, or nonionic. Surfactants in the poloxamer and poloaxamines family are commonly used in particle synthesis. Surfactants that may be used, include, but are not limited to PEG, Tween-80, gelatin, dextran, pluronic L-63, PVA, methylcellulose, lecithin, DMAB and PEMA. Additionally, biodegradable and biocompatible surfactants including, but not limited to, vitamin E TPGS (D-α-tocopheryl polyethylene glycol 1000 succinate). In certain embodiments, two surfactants are used. For example, if the particle is produced by a double emulsion method, the two surfactants can include a hydrophobic surfactant for the first emulsion, and a hydrophobic surfactant for the second emulsion.

Therapeutic formulations of the polypeptide binding agent are prepared for storage by mixing the polypeptide binding agent having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, succinate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; or metal complexes (e.g., Zn-protein complexes).

### TIMP-GLIA Particle

Tolerogenic Immune Modifying Particles (TIMP) - Gliadin ("TIMP-GLIA") is a first-in-class, nonimmunosuppressive agent to specifically inactivate, or tolerize, gliadin-specific T cells, thereby abrogating and/or reversing the underlying pathology of CD. TIMP-GLIA may also be thought of as a noninfectious disease therapeutic vaccine or "inverse vaccine" (Steinman 2010).

TIMP-GLIA is comprised of gliadin extract drug substance within a negatively charged polymer matrix of PLGA (Poly(DL-lactide-coglycolide)) particles. TIMP deliver the gliadin antigen via natural phagocytosis of the PLGA particles, a noninflammatory process. It is hypothesized that this phagocytosis of the particles leads to antigen presentation of the gliadin by antigen presenting cells (APCs) primarily in the spleen and liver. The gliadin specific T cells become anergic, or are deleted, or switch to T regulatory cells, thereby tolerizing the immune system to the gliadin antigen, and eliminating the deleterious immune cascade typically initiated by the gliadin specific T cells in response to gliadin. TIMP-GLIA is designed and directed to specifically address and abrogate the T cell response that drives CD. (Getts 2015).

Exemplary gliadin antigens are disclosed in U.S Patent 9,616,113. Discontinuous epitopes of gliadin (Protein-glutamine gamma-glutamyltransferase 2) include D151, E153, E154, E155, E158, D306, N308, N310; SEQ ID NO: 1725 D434, E435, E437, D438; E329; E153; R19, E153, M659; OR C277, H335, D358. Additional proteins or peptides that induce gluten sensitivity are disclosed in WO 2010/060155, set out in SEQ ID NOs: 631-1116. For example, "gluten" or "gluten protein" encompasses alpha (α), beta (β), gamma (γ) and omega (ω) gliadins, and low and high molecular weight (LMW and HMW) glutenins in wheat, B, C and D hordeins in barley, β, γ and CO secalins in rye, and optionally avenins in oats. "Gluten peptides" are peptides derived from, or encompassed within, one or more of the gluten proteins. Gliadin refers to the aqueous alcohol-soluble fraction of gluten, particularly, but not exclusively, gluten derived from wheat, for example *Triticum aestivum.* Glutenin refers to the aqueous alcohol-insoluble fraction of gluten, particularly but not exclusively, gluten derived from wheat, for example *Triticum aestivum.* Hordein refers to gluten derived from barley, *Hordein vulgare.* Secalin refers to gluten derived from rye, *Secale cerale.* Avedin refers to gluten derived from oats, *Avena sativa.* Exemplary antigenic sequences are set out in SEQ ID NOs: 1 to 1116 of WO 2010/060155.

In various embodiments, the particle is administered at a dose from about 0.1 to about 10 mg/kg. In various embodiments, the TIMP-GLIA is administered at a dose of about 0.1 mg/kg, 0.5 mg/kg, 1.0 mg/kg, 2.0 mg/kg, 4.0 mg/kg, 8.0 mg/kg or 10 mg/kg. In various embodiments, the TIMP-GLIA is administered at a dose of about 8.0 mg, 80 mg, 320 mg, 640 mg or 800 mg. Also contemplated are values within and between the recited dose endpoints.

Splenic and liver APC populations express numerous scavenger receptors which during homeostasis play an important role in the recognition and recycling of dying leukocytes, red blood cells, and other debris on a daily basis. Targeting of and tolerogenic stimulation of APCs is crucial for immune tolerance induction. Importantly, these activities occur without triggering inflammation or breaking peripheral immune tolerance. TIMP have been designed to take advantage of this targeting pathway.

In vitro studies have shown that TIMP-GLIA does not cause T cell mitogenicity. TIMP-GLIA does not interact directly with the T cell receptor (TCR) nor does it cause activation of T cells through binding of co-stimulation molecules expressed on T cells, platelets and other leukocytes. TIMP-GLIA regulates gliadin specific T cells indirectly, through antigen presenting cells.

All previously attempted immune tolerance strategies either using monoclonal antibodies (i.e., anti-CD3, cytotoxic T-lymphocyte antigen 4 [CTLA-4]) or small molecules (rapamycin) have not exploited this pathway. It is hypothesized that the TIMP mechanism of action depends upon uptake of particles by APCs, particularly splenic marginal zone macrophages expressing scavenger receptors, such as the MAcrophage Receptor with COllagenous structure (MARCO), in a fashion similar to the clearance of apoptotic debris. Studies using particles or apoptotic cells have shown that treatment induced production of the regulatory cytokines interleukin-10 (IL-10) and transforming growth factor beta (TGF-β) in the spleen, as well as upregulation of inhibitory ligands on macrophages, such as programmed death-1 (PD-1). Downstream events include the induction of T cell anergy and the activation of regulatory T cells. The overall result is abrogation of pro-inflammatory T cell activity, reduction of leukocyte accumulation in tissues, and importantly, disappearance of signs of disease (Getts 2015).

The administration of the TIMP-GLIA particle improves one or more signs or symptoms of Celiac Disease or gluten sensitivity. Exemplary symptoms, include, but are not limited to the one or more symptoms is selected from the group consisting of weight loss, fatigue, headache, iron deficiency, folic acid deficiency, vitamin D deficiency, vitamin B12 deficiency, villous atrophy, intestinal mucosal damage, and low bone density. See e.g., Woodward, Clin Exp Gastroenterol. 2016; 9: 225-236.. One or more symptoms include subjective symptoms such as villous atrophy, tetramer staining, ELISPOT, miRNA, Exosomes, RNA, and DNA.

The disclosure contemplates modulation of tolerance by modulating Th1 response, Th2 response, Th17 response, or a combination of these responses. Modulating Th1 response encompasses changing expression of, e.g., interferon-gamma. Modulating Th2 response encompasses changing expression of, e.g., any combination of IL-4, IL-5, IL-10, and IL-13. Typically an increase (decrease) in Th2 response will comprise an increase (decrease) in expression of at least one of IL-4, IL-5, IL-10, or IL-13; more typically an increase (decrease) in Th2 response will comprise an increase in expression of at least two of IL-4, IL-5, IL-10, or IL-13, most typically an increase (decrease) in Th2 response will comprise an increase in at least three of IL-4, IL-5, IL-10, or IL-13, while ideally an increase (decrease) in Th2 response will comprise an increase (decrease) in expression of all of IL-4, IL-5, IL-10, and IL-13. Modulating Th17 encompasses changing expression of, e.g., TGF-β, IL-6, IL-21 and IL-23, and effects levels of IL-17, IL-21 and IL-22.

### EXAMPLES

### Example 1-Protocol for First in Human Dosing of TIMP-GLIA

Previous toxicology studies in animals showed that TIMP-GLIA dosages used during the non-GLP study were 0, 10, 50, 75, 100, and 200 mg/kg (HED: 0, 1.6, 8, 12, 16, and 32 mg/kg) and administered IV on Days 1 and 8, with the necropsy being on Day 15 or Day 17 depending upon tissue/blood sampling requirements. In summary, TIMP-GLIA produced no significant toxicological or pathological effects at 10 mg/kg when administered IV on Days 1 and 8. A number of clinical pathology and microscopic histopathological effects were seen at dosages of > 50 mg/kg, predominately in the liver and the spleen. At 200 mg/kg two animals were euthanized due to drug-related effects.

TIMP-GLIA that had undergone the product purification mentioned above was administered IV to rats (10/sex/group) at dosages of 0, 4, 10, 50, and 75 mg/kg (HED: 0, 0.64, 1.6, 8, and 12 mg/kg). Infusions occurred on Days 1, 8, and 15 with the necropsy being on Day 16. An additional 5 rats/sex/group underwent the same dosing regimen followed by a 28-day recovery period after the last dose and necropsy on Day 43. Another 5 rats/sex/group were dosed on Day 1 and Day 8 and euthanized on Day 11 for pathology investigations and groups of 12 rats/sex/TIMP-GLIA group plus 6 /sex/control group were dosed on Days 1 and 8 for measurement of TGLIA-concentrations over time for TK analysis and for cytokine measurement. The animals in the TK/cytokine group were euthanized after the last blood draw.

All animals survived until the scheduled necropsy and remained in good health throughout the course of the study. There were no significant abnormal clinical findings during the study and no drug-related effects were noted on body weight, body weight gain, food consumption, ophthalmology exams, physical exams, clinical observations, functional observational battery, body temperature and serum cytokine levels. The results of the microscopic evaluation of the tissues obtained at necropsy of the animals terminated on Day 16 or Day 43 from each sex and dose group did not exhibit any pathologically significant findings.

The present disclosure provides a Phase 1, first-in-human (FIH), 2-part, multicenter study to assess the safety, tolerability and PK of TIMP-GLIA in subjects with CD and healthy subjects.

Part A is a traditional, open-label, single ascending dose (SAD) study with staggered dosing. The dose escalation and cohort size in Part A are based upon accepted methodology for phase I studies (Le Tourneau 2009, Rubinstein 2003). Part A includes an accelerated titration 2+2 and traditional 3 + 3 design with rapid dose escalation in each population (CD and healthy subjects).

Successive cohorts of each population will receive a single dose of TIMP-GLIA. Decisions regarding the progression or termination of dosing will be made separately for each population (CD or healthy subjects) based upon the respective safety data.

Eligible subjects (at least 19 CD subjects and at least 19 healthy subjects) will be enrolled into escalating dose cohorts (n = 2/cohort for 2 dose levels followed by n = 3/cohort for 5 dose levels). TIMP-GLIA will be administered as a single IV infusion on Day 1. A staggered dosing strategy is used in Part A.

For subjects with CD, at least 168 hours (7 days) will elapse prior to dosing the next subject. Adverse events (AEs), vital signs, pulse oximetry, and electrocardiograms (ECGs) and laboratory data (serum chemistry, coagulation, hematology and urinalysis, cytokines) from samples obtained through at least 24 hours post dose will be assessed before dosing the subsequent subject(s).

For healthy subjects, at least 48 hours will elapse prior to dosing the next subject. This corresponds to the time when subjects would be discharged from the clinic, provided extended medical supervision is not required in the opinion of the investigator. Adverse events (AEs), vital signs, pulse oximetry, and electrocardiograms (ECGs) and laboratory data (serum chemistry, coagulation, hematology and urinalysis) from samples obtained through at least 24 hours post dose will be assessed before dosing the subsequent subject(s).

Duration of the study investigational period for CD subjects is up to 91 days (screening up to 28 days + 1 treatment day + follow-up at Day 60 +/- 3 days). In addition, telephone follow-up by a health care practitioner is required after the Day 60 outpatient visit (i.e. Day 90, Day 120 and Day 180, all +/- 3 days). In one embodiment, the duration of the study may be up to 73 days for a single dose recipient (i.e., screening period up to 28 days + investigational period up to 45 days [Day 42 +/- 3 days]).

In another embodiment, total duration of the study investigational period for healthy subjects is up to 91 days (screening up to 28 days + 1 treatment day + follow-up at Day 60 +/- 3 days).

Part B will follow as a repeat dose design using the dose level selected from Part A. Consenting subjects will be screened within 28 days (Day -28 to -1) prior to admission to the clinical research unit on Day -1 for baseline assessments.

Part B will be a repeat-dose study with 3 subjects who will receive the first infusion on Day 1 and the second infusion on Day 8. The dose selection for Part B will be based on the emerging safety and tolerability data. The first subject in Part B will be observed for at least 48 hours after the second dose before dosing of remaining subjects in Part B. For subjects with CD, at least 168 hours (7 days) will elapse after the second dose and safety confirmed before the next subject will be dosed. For healthy subjects, at least 48 hours will elapse after the second dose and safety confirmed prior to dosing the next subject.

Thereafter, dosing of the subjects in Part B can occur on the same day or over multiple days across the participating clinical sites to meet operational needs. If a dose-limiting toxicity occurs in 1 of the 3 subjects in Part B, the Safety Committee may decide to enroll an additional 3 subjects at the same dose to confirm ambiguous safety or tolerability findings. Depending upon the emerging safety data, they may also recommend enrollment of an additional cohort of 3 subjects at a lower dose to discern the safety profile of repeat dosing. Repeat dosing will provide support for safe and tolerable dosing using the 2-dose regimen to be investigated in the future proof-of-concept (POC) clinical trial.

Part B total dosing: Total duration of the study investigational period for CD subjects is up to 91 days (screening up to 28 days + 2 treatment days, 7 days apart + follow-up at 60 +/- 3 days after last dose). In addition, telephone follow-up by a health care practitioner is required after the Day 60 outpatient visit (i.e., Day 90, Day 120, Day 180, all +/- 3 days). In one embodiment, for a repeat dose recipient the total duration of the study is up to 80 days (i.e., screening period up to 28 days + investigational period up to 52 days [Day 49 +/- 3 days]).

Part B: Total duration of the study investigational period for healthy subjects is up to 91 days (screening up to 28 days + 2 treatment days, 7 days apart + follow-up at 60 +/- 3 days after last dose).

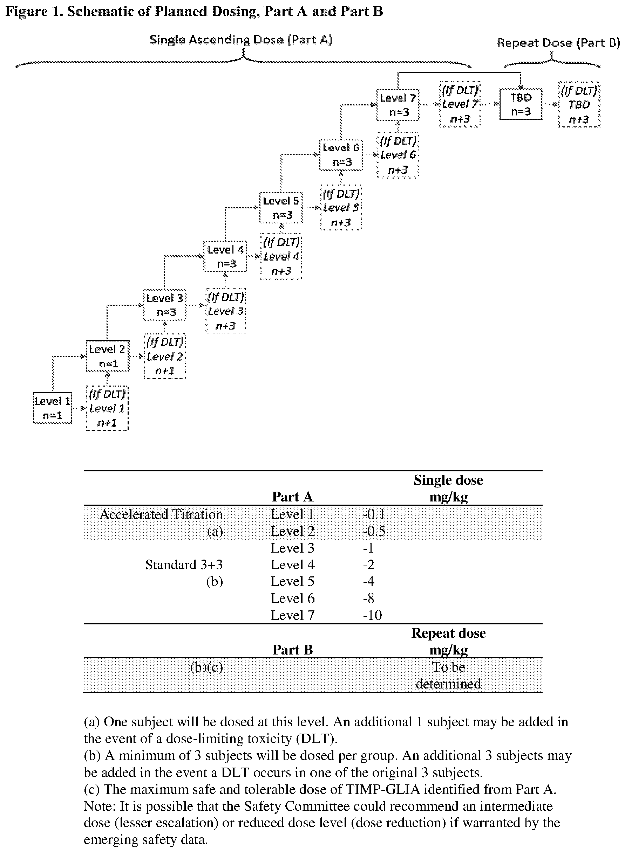

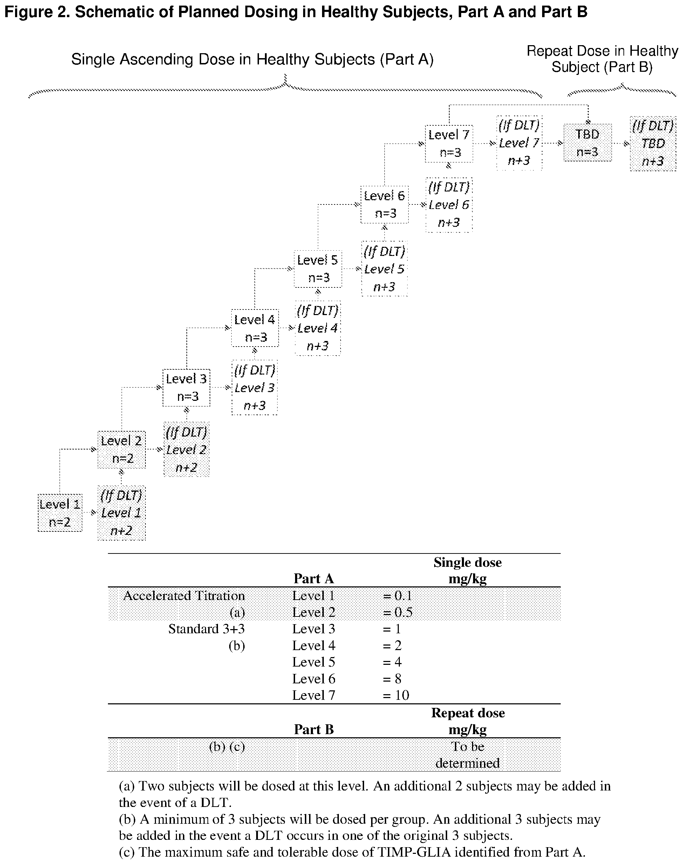

Starting Dose: A starting dose of 0.1 mg/kg followed by 0.5 mg/kg will be explored in 1 subject each via an accelerated titration 1+1 schedule. This is followed by dosing cohorts of 3 subjects according to a standard 3+3 escalation schedule beginning with a dose of 1 mg/kg. The larger cohorts are employed at 1 mg/kg when pharmacologic effects are more likely to be observable. Planned doses and rationale is listed below in Table 1 and Figures 1 and 2.

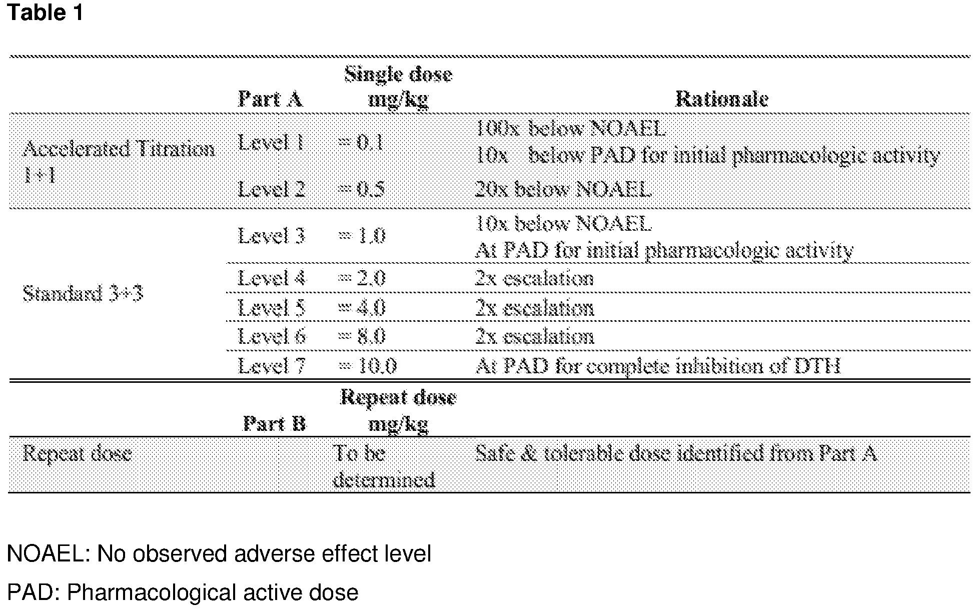

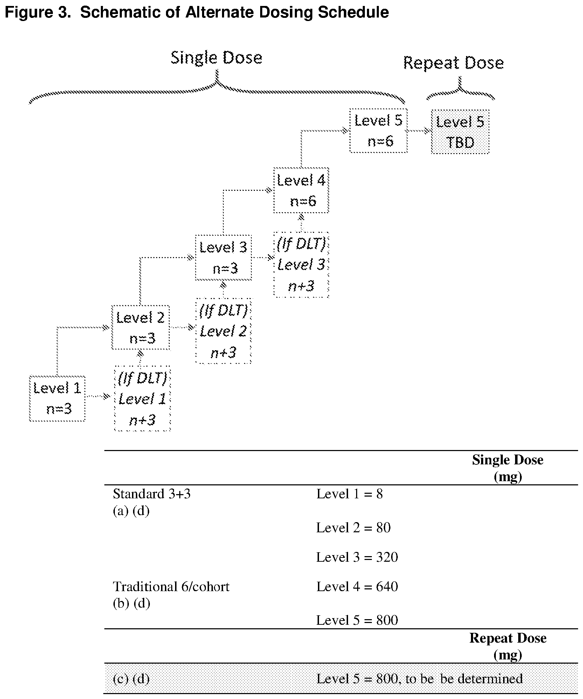

In an alternate dosing schedule, subjects (healthy or Celiac) may be administered TIMP-GLIA at 8 mg, 80 mg, 320 mg, 640 mg, or 800 mg, administered by IV infusion (Figure 3). A 8.0 mg starting level is approximately equal to 0.1 mg/kg, e.g., for an 80 kg subject. Dose escalation at this levels is set out in Table 2.

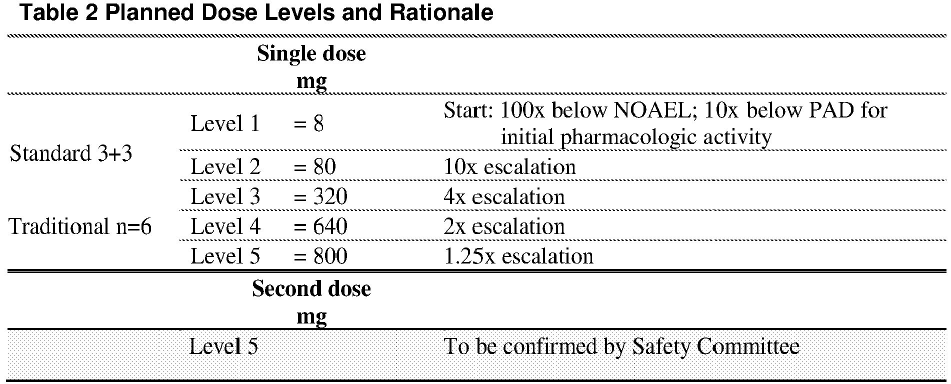

PRIMARY ENDPOINTS: Safety will be characterized by incidence, severity, and reversibility of AEs, physical examination findings, 12-lead ECG results, arterial oxygen saturation levels by pulse oximetry, vital signs, measurements, anti-gliadin antibody (i.e., anti-drug antibody), routine clinical laboratory test results (hematology, serum chemistry, coagulation, urinalysis) and specialized laboratory test results (e.g., acute phase cytokines; additional vascular / thrombotic markers; mast cell activation via tryptase; complement markers, peripheral blood T-cell proliferation) if clinically indicated by the appearance of systemic symptoms of anaphylaxis/analphylactoid reaction/cytokine release syndrome or IRR.

The analysis of samples drawn for cytokine testing (TNF-α, IFN-γ, IL-2, IL-6, IL-4, IL-5, IFN-δ, IL-8, IL-10, GM-CSF, MIP1α, MIP1β, GRO α, IFNα, fractaline, IP-10, IL-1α,IL-1β, EGF and other cytokines) are performed. If the symptoms do not emerge, the scheduled predose and 8-hour to 24-hour post dose samples for cytokine testing can be analyzed at the end of the trial unless otherwise specified by the medical monitor, sponsor, or Safety Committee or as necessary for compliance with the established sample stability. If peripheral blood T-cell proliferation in response to ex vivo stimulation with gliadin following exposure to TIMP-GLIA assay is performed, results will be reported at the end of the study or as otherwise specified by the sponsor, medical monitor, or Safety Committee. Tolerability will be characterized by extent of dose escalation attained without dose limiting toxicity.

SECONDARY ENDPOINTS: PK of TIMP-GLIA will be derived from the plasma TIMP-GLIA concentration - time curve by noncompartmental analysis. The primary PK parameters are maximal observed concentration (Cₘₐₓ), last measurable concentration (Cₗₐₛₜ), time of maximal observed concentration (Tₘₐₓ), and area under the curve from time zero and extrapolated to infinity (AUC_{inf}) and area under the concentration-time curve from time zero to time of the last measurable concentration (AUCₗₐₛₜ).

Other PK variables will be derived if feasible: terminal elimination half-life (t_{½}), area under the curve over the dosing interval (AUC_{τ}), area under the curve from time zero (time of dosing) to time t, where a relevant twill be determined based upon the observed data (AUCₜ), total body clearance (CL), volume of distribution (V_{d}), and accumulation index (R_{acc}). The terminal half-life will be calculated as t½ = ln(2)/λ_{z}, where λ_{z} is the terminal rate constant. λ_{z} will be estimated as the slope from a linear regression with the natural logarithm to the concentration as the response variable, and time as the explanatory variable. Valid observations from the final part of the curve, which is approximately linear, will be used for the analysis.

Additionally, the study will assess titers of circulating anti-gliadin antibodies to TIMP-GLIA (i.e., anti-drug antibodies) on study Day 14, Day 30 (Part A) or 38 (Part B), and Day 60.

SUBJECTS: A subject has celiac disease characterized as follows:
a. The subject has a history of biopsy-confirmed celiac disease (intestinal histology showing villous atrophy) according to expert guidelines current at the time of diagnosis; and
b. The subject is positive for HLA-DQ2.5 (HLA-DQA1 *0501 / B1*0201) or HLA-DQ8.1 (DQA1*0301/B1*0302). Phenotyping for HLA-DQ2.5 (HLA-DQA1*0501 / B1*0201) and HLA-DQ8.1 (DQA1*0301/B1 *0302) are performed by a bioanalytical laboratory and may be assessed at Screening Visit if not previously done or unknown;
c. The subject has no known gluten exposure for approximately 2 months prior to enrollment and is willing to maintain a gluten-free diet for the duration of the study;
d. The subject has a total immunoglobulin A (IgA) titer within normal limits or has partial IgA deficiency (-5% of celiac patients) defined by a reduced serum IgA level of 3 - 70 mg/dL at screening and the subject has negative or weak positive recombinant human transglutaminase (tTG)-specific IgA titer at screening or for a subject with selective IgA deficiency (-2% of celiac patients), deamidated gliadin peptide (DGP)-specific IgG titer is negative or weak positive at screening. Total serum Immunoglobulin A (IgA), tissue transglutaminase (tTG)-specific IgA antibody or deamidated gliadin peptide (DGP)-specific IgG antibody will be measured at a bioanalytical laboratory.

A healthy subject (n=at least 22) is an adult man or women, 18 to 65 years of age, inclusive, at Screening Visit. The healthy subject has a body mass index (BMI) that is >16 kg/m² with a minimum body weight of 33 kg up to a maximum body weight of 129 kg, inclusive, (or alternatively has a minimum body weight >45 kg (99.0 lb) up to a maximum body weight of 121 kg (266.2 lb) at Screening Visit and if BMI <18 ("underweight") or >25 ("overweight" or "obese"), is otherwise healthy in the opinion of the investigator. Healthy subjects are also within specified ranges of clinical laboratory tests as required by the study criteria.

TIMP-GLIA: For this study, TIMP-GLIA is comprised of gliadin extract within a negatively charged (-35mV to -50mV) polymer matrix of PLGA particles with an average size between 400 nm - 800 nm and approximate size distribution between ~200 nm and ~700 nm. There is ~10 µg of refined gliadin per mg of PLGA particles.

TIMP-GLIA is supplied as a lyophilized powder in a single-use, 20-mL glass vial containing approximately 1 mg refined gliadin and 100 mg of PLGA particles. TIMP-GLIA is to be reconstituted with 2.5 mL of Sterile Water for Injection (SWI), and further diluted for infusion with 0.9 % Sodium Chloride Injection, USP. The drug solution is administered by IV infusion using a controlled infusion device on the day of preparation. Specific reconstitution and dilution and storage instructions are provided in the Pharmacy Manual. TIMP-GLIA vials are stored at 2° C to 8° C (36° to 46° F) and protected from light in a secure, temperature-monitored, limited access location.

Dosing and Regimen: Initially, TIMP-GLIA is administered once as an IV infusion by a controlled infusion device. The planned doses will range as shown in Figure 1, Figure 2 and Figure 3. Dosing will occur on Day 1 in Part A and on Day 1 and Day 8 in Part B (repeat dosing) (7 days apart). For example, in Part A, subjects are dosed at 0.1 mg/kg, 0.5 mg/kg and 1 mg/kg in a single iv infusion. In certain study cohorts, a starting dose of 0.1 mg/kg followed by 0.5 mg/kg will be explored in 1 subject each via an accelerated titration 1+1 schedule. This is followed by dosing cohorts of 3 subjects according to a standard 3+3 escalation schedule beginning with a dose of 1 mg/kg. For the 3+3 infusion, 3 subjects may be dosed at each of 1.0 mg/kg, 2.0 mg/kg, 4.0 mg/kg, 6.0 mg/kg, or 8.0 mg/kg, and an additional 1 to 3 subjects may be added to the groups if there is a drop out event. Subjects may also be dosed according to this schedule using the doses as set out in Figure 3.

As a safety precaution venous access should be maintained for emergency use for 24 hours after dosing. On dosing days, subjects will receive the assigned study drug after the subject has fasted from food and beverage approximately 2 hours prior to infusion start, and 1 hour after infusion end. Water may be provided ad libidum.

After all subjects at a dose level have completed study procedures through Day 3 (48 hours post dose), the overall safety and tolerability of the dose is determined by Safety Committee evaluation of the safety data [Adverse Events (AEs), vital signs, 12-lead ECGs, clinical laboratory tests] for the current cohort and available cumulative AE data from previous cohorts against the Stopping Rules.

Dose reduction: Depending upon emerging safety data, the Safety Committee may recommend dosing a cohort of 1 subject (accelerated titration 1+1, Part A) or 3 subjects (standard 3+3, Part A and Part B) at a dose lower than the planned level to better identify the maximum tolerable dose of TIMP-GLIA.

Blood samples are collected via a peripherally placed IV cannula or by direct venipuncture in a suitable forearm vein. Samples for clinical chemistry will be collected under fasting conditions and in accordance with acceptable laboratory procedures/protocols.

### Analytical Methods

*Mast Cell Activation and Complement Activation and Supplementary Vascular*/*Thrombotic Assessment*: Symptoms of anaphylaxis/anaphylactoid response or IRR (e.g., fever, nausea, chills, rigors, hypotension, tachycardia, asthenia, headache, rash, scratchy throat, and dyspnea) are followed up with additional laboratory testing for mast cell activation (Tryptase), complement activation (C3a, C5a,CH50, C5-9), and for vascular/thrombotic markers (D-dimer, vWG, ICAM-1, Fibrinogen, Prothrombin Fragment 1,2 and P-selectin). Predose samples will be obtained from all subjects, with additional post dose samples to be taken from symptomatic subjects *pro re nata* (prn)if clinically indicated. Samples should be analyzed promptly and results are to be reported to the investigator as soon as possible in the event of clinical presentation of anaphylaxis/anaphylactoid response or IRR.

*Whole Blood for PSMC Isolation*: Samples are obtained from all subjects predose. Additional sample(s) will be collected prn if clinically indicated by appearance of symptoms of anaphylaxis/anaphylactoid response/cytokine release syndrome or IRR. Peripheral blood mononuclear cells (PBMCs) will be isolated from whole blood samples if clinically indicated to determine the number of IFN-γ and IL-10 producing T cells by enzyme-linked immunospot (ELISpot) assay and for T cell proliferation in response to ex vivo gliadin stimulation (by an established proliferation assay). Results are analyzed only if clinically indicated or at the discretion of the sponsor and available at the end of the trial or as necessary for compliance with the established sample stability.

Cytokine Analysis: Samples for determination of acute phase cytokines (TNF-α, IFN-γ, IL-2, IL-6, IL-4, IL-5, IFN-δ, IL-8, IL-10, GM-CSF, MIP1α, MIP1β, GRO α, IFNα, fractaline, IP-10, IL-1α,IL-1β,or other cytokines) are to be collected predose and through 24 hours post dose, e.g., 8 hour and 24 hour, (for all subjects). Additional samples are to be obtained prn from subjects exhibiting systemic symptoms of anaphylaxis/analphylactoid reaction/cytokine release syndrome or IRR such as fever, nausea, chills, rigors, hypotension, tachycardia, asthenia, headache, rash, scratchy throat, and dyspnea. Measurement of anti-gliadin antibodies (i.e., anti-drug antibodies) and immune complex detection by C1q binding and Raji cell assay in serum will be performed using validated methodology at appropriate bioanalytical laboratory(ies). For example, deamidated gliadin peptide (DGP)-specific IgG antibody will be used as an anti-drug antibody test.

### Example 2

### Safety and Tolerability Assessment from First in Human Dosing of TIMP-GLIA

In the present study, the criteria set out above in Example 1 for conducting the study was used with changes noted below.

For inclusion criteria, the patient was an adult man or women, 18 to 75 years of age, inclusive, at Screening Visit. The subject has a body mass index (BMI) that is >16 kg/m² with a minimum body weight of 33 kg up to a maximum body weight of 129 kg, inclusive, at Screening Visit and if BMI <18 ("underweight") or >25 ("overweight" or "obese"), is otherwise healthy in the opinion of the investigator.

The CD patient is not required to be genotyped, but biopsy confirmed CD was sufficient. Also, the subject had no known gluten exposure for at least 10 days prior to the Screening Visit and maintained a gluten-free diet for the duration of the study.

STUDY: To establish the safety, tolerability and pharmacokinetics (PK) of TIMP-GLIA in humans, 23 subjects with biopsy proven celiac disease (CD) were enrolled in a 2-part Phase 1 multi-center study. Part A of the study evaluated single ascending doses of TIMP-GLIA; Part B evaluated repeat (Days 1 and 8) ascending doses of TIMP-GLIA. Consenting subjects were screened for eligibility within 28 days of Day 1/ Dose 1. Subjects were confined to a clinical study unit for 12 hours prior to dosing and for 48 hours following dosing. All subjects were followed for safety through Day 180. Safety and tolerability were assessed by physical exam, electrocardiogram, telemetry, pulse oximetry, vital signs, adverse events (AEs), serum chemistries, complete blood count with differential, serum cytokines and chemokines, plasma complement levels, gliadin-specific T cell proliferation and cytokine secretion, and deamidated gliadin peptide (DGP) immune globulin G (IgG). PK was measured pre-dose, 30 minutes, 35 minutes, end of infusion, 1, 4, 12, 24, 48, and 144 hours after each dose. Safety was monitored throughout the trial by an independent Data Monitoring Committee.

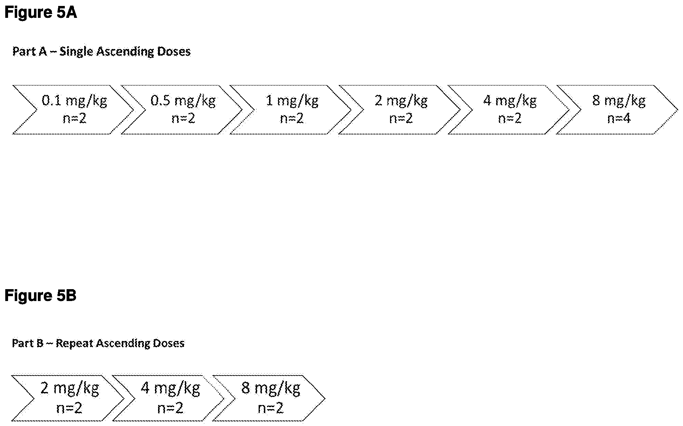

In Part A of the study, 17 subjects were administered a single intravenous (IV) dose of TIMP-GLIA, ranging from 0.5 mg/kg to 8 mg/kg (Figure 5A). TIMP-GLIA was infused over 30 minutes, except in 2 subjects at 8 mg/kg where it was infused over ~2.5 hours. Subjects were dosed at least 7 days apart, once safety and tolerability was confirmed in the prior subject. Dose escalation proceeded when safety and tolerability was confirmed in the prior dose level.

In Part B of the study, 6 subjects were administered 2 IV doses of TIMP-GLIA ranging from 2 to 8 mg/kg up to a maximum of 650 mg (Figure 5B) on Days 1 and 8. TIMP-GLIA was infused over ~2.5 hours. Dose escalation proceeded when safety and tolerability was confirmed in the prior dose level.

### RESULTS:

*Adverse Events*: Evaluation of patients in Part A and Part B of the study revealed that TIMP-GLIA is safe and tolerable up to a dose of 8 mg/kg. During Part A of the study, 2 of the 4 subjects who received a single 8 mg/kg dose experienced a mild to moderate infusion reaction (IR). The first subject developed a rash (grade 2), systolic hypotension (grade 2), and concentration disturbance (grade 1) at ~5 minutes into the infusion. The investigator discontinued the infusion and the symptoms resolved within minutes. The second subject experienced flushing (grade 2), nausea (grade 1), vomiting (grade 1), back pain (grade 1), and visual changes (grade 1). The investigator interrupted the infusion and treated the subject with diphenhydramine. The symptoms resolved within minutes and the infusion was restarted and completed without further incident.

Upon review of all safety data from Part A, the Data Monitoring Committee recommended that 1) the TIMP-GLIA particle diluted in 200 mL of normal saline (originally 100 mL), and 2) the particle be infused over ~2.5 hours - 20mL/hour for the first 15 minutes, 40 mL/hour for the next 15 minutes, and then 80 mL/hour for the duration of the infusion.

During Part B of the study, 1 subject (of 2) receiving 4 mg/kg experienced mild back pain during both infusions at ~10 minutes into the infusion. The investigator briefly interrupted the first infusion and did not interrupt the second infusion. There were no other AEs reported in any of the other 5 subjects receiving a repeat dose of TIMP-GLIA.

There were no serious AEs (SAEs) reported in any subject in Part A or Part B. All but 1 AE were ≤ grade 2 (moderate); 1 subject reported grade 3 (non-celiac) colitis that the investigator deemed not related to TIMP-GLIA. The most frequent events observed in ≥ 2 subjects include: flushing (n=5, 22%), headache (n=4, 17%), back pain (n=3, 13%), fatigue (n=2, 9%), abdominal pain (n=2, 9%), and diarrhea (n=2, 9%). Flushing was only observed in the single dose subjects. Two subjects in Part A experienced back pain (1 at 4 mg/kg, 1 at 8 mg/kg) compared to 1 subject (4 mg/kg) in the repeat dose group. There were no trends for any other AE.

*Serum Cytokines and Chemokines*: Levels of the following cytokines and chemokines were measured by ELISA: EGF, fractalkine, GM-CSF, GRO α, IFN-α, IFN-γ, IL-1α, IL-1β, IL-2 soluble receptor, IL-4, IL-5, IL-6, IL-8, IL-10, IL-12, IL-13, IL-17, IP-10, MIP1α, MIP1β and TNF-α). Samples were taken in both Parts A and B at: pre-dose, 8 and 24 hours, 7 and 14 days after each dose. There were no clinically significant changes in any serum cytokines or chemokines, including the inflammatory cytokines through 14 days following each dose.

*Gliadin-specific ex vivo T-cell proliferation and cytokine release*: Whole blood was collected from patients in both Part A (4 and 8 mg/kg dose groups only) and Part B for the isolation of peripheral blood monocytes (PBMCs). Whole blood was collected pre-dose and 7 days after each dose. Antigen-specific (gliadin in this case) ex vivo T-cell proliferation and cytokine release assays (IFN-γ, IL-1β, IL-2, IL-4, IL-6, IL-8, IL-10, IL-12p70, IL-13, TNF-α) were performed using standardized and validated assays. Briefly, PBMCs isolated from each subject were plated at a concentration of 100,000 cells/well in a multi-well assay plate and stimulated with culture media alone (negative control), anti-CD3 (positive control), CEFT MHC-II pooled peptides (positive control) or Gliadin Immunodominant peptides (alpha gliadin - QLQPFPQPELPYPQPQS (SEQ ID NO: 1) or omega gliadin- PFPQPEQPFPW (SEQ ID NO: 2)). After 48-72 hours of incubation, culture supernatants were collected for cytokine analysis using Mesoscale Discovery (MSD) multiplexed cytokine plates. T-cell proliferation was measured using a luminescence-based assay using Promega's CellTiterGlo^{®} assay reagent.

There were no changes in T-cell proliferation or cytokine levels observed in any dose group. Tables 3A-D below show change from baseline for Part B subjects for T cell proliferation (Table 3A) and inflammatory cytokines IFN-γ (Table 3B), IL-4 (Table 3C), and IL-6 (Table 3D) by dose group.

**Table 3A**

| **Summary Statistics of Observed Values and Change from Baseline Values - T Cell Proliferation (A.U)** | | | | | | | | | | | | | |
|---|---|---|---|---|---|---|---|---|---|---|---|---|---|
| | | **Observed Values** | | | | | | **Change from Baseline** | | | | | |
| **Treatment** | **Day** | **N** | **Mean** | **SD** | **Min** | **Median** | **Max** | **N** | **Mean** | **SD** | **Min** | **Media n** | **Max** |
| 2.0 mg/kg | Day 1 | 2 | 1.0000 | 0.00000 | 1.000 | 1.0000 | 1.000 | | | | | | |
| | Day 8 | 2 | 0.9500 | 0.07071 | 0.900 | 0.9500 | 1.000 | 2 | -0.0500 | 0.07071 | -0.100 | -0.0500 | 0.000 |
| | Day 14 | 1 | 1.0000 | NA | 1.000 | 1.0000 | 1.000 | 1 | 0.0000 | NA | 0.000 | 0.0000 | 0.000 |
| | | | | | | | | | | | | | |
| 4.0 mg/kg | Day 1 | 2 | 0.9000 | 0.00000 | 0.900 | 0.9000 | 0.900 | | | | | | |
| | Day 8 | 2 | 1.0000 | 0.00000 | 1.000 | 1.0000 | 1.000 | 2 | 0.1000 | 0.00000 | 0.100 | 0.1000 | 0.100 |
| | Day 14 | 2 | 0.9500 | 0.07071 | 0.900 | 0.9500 | 1.000 | 2 | 0.0500 | 0.07071 | 0.000 | 0.0500 | 0.100 |
| | | | | | | | | | | | | | |
| 8.0 mg/kg | Day 1 | 2 | 0.9500 | 0.07071 | 0.900 | 0.9500 | 1.000 | | | | | | |
| | Day 8 | 2 | 1.0000 | 0.00000 | 1.000 | 1.0000 | 1.000 | 2 | 0.0500 | 0.07071 | 0.000 | 0.0500 | 0.100 |
| | Day 14 | 2 | 1.0000 | 0.00000 | 1.000 | 1.0000 | 1.000 | 2 | 0.0500 | 0.07071 | 0.000 | 0.0500 | 0.100 |
| Day 1 and Day 8 are pre-dose levels | | | | | | | | | | | | | |

**Table 3B**

| **Summary Statistics of Observed Values and Change from Baseline Values - Interferon Gamma (pg/mL)** | | | | | | | | | | | | | |
|---|---|---|---|---|---|---|---|---|---|---|---|---|---|
| | | **Observed Values** | | | | | | **Change from Baseline** | | | | | |
| **Treatment** | **Day** | **N** | **Mean** | **SD** | **Min** | **Median** | **Max** | **N** | **Mean** | **SD** | **Min** | **Media n** | **Max** |
| 2.0 mg/kg | Day 1 | 2 | 5.8000 | 6.36396 | 1.300 | 5.8000 | 10.300 | | | | | | |
| | Day 8 | 2 | 7.2000 | 4.80833 | 3.800 | 7.2000 | 10.600 | 2 | 1.4000 | 1.55563 | 0.300 | 1.4000 | 2.500 |
| | Day 14 | 1 | 11.600 | NA | 11.600 | 11.600 0 | 11.600 | 1 | 10.3000 | NA | 10.300 | 10.300 0 | 10.300 |
| | | | | | | | | | | | | | |
| 4.0 mg/kg | Day 1 | 2 | 9.8500 | 9.12168 | 3.400 | 9.8500 | 16.300 | | | | | | |
| | Day 8 | 2 | 15.400 | 16.9705 | 3.400 | 15.400 0 | 27.400 | 2 | 5.5500 | 7.84889 | 0.000 | 5.5500 | 11.100 |
| | Day 14 | 2 | 14.900 | 15.4149 | 4.000 | 14.900 0 | 25.800 | 2 | 5.0500 | 6.29325 | 0.600 | 5.0500 | 9.500 |
| | | | | | | | | | | | | | |
| 8.0 mg/kg | Day 1 | 2 | 13.000 | 5.37401 | 9.200 | 13.000 0 | 16.800 | | | | | | |
| | Day 8 | 2 | 9.300 | 4.66690 | 6.000 | 9.3000 | 12.600 | 2 | -3.7000 | 0.70711 | -4.200 | -3.7000 | -3.200 |
| | Day 14 | 2 | 11.400 | 1.41421 | 10.400 | 11.400 0 | 12.400 | 2 | -1.6000 | 6.78823 | -6.400 | -1.6000 | 3.200 |
| Day 1 and Day 8 are pre-dose levels | | | | | | | | | | | | | |

**Table 3C**

| **Summary Statistics of Observed Values and Change from Baseline Values b - Interleukin 4 (pg/mL)** | | | | | | | | | | | | | |
|---|---|---|---|---|---|---|---|---|---|---|---|---|---|
| | **Observed Values** | | | | | | | **Change from Baseline** | | | | | |
| **Treatment** | **Day** | **N** | **Mean** | **SD** | **Min** | **Median** | **Max** | **N** | **Mean** | **SD** | **Min** | **Media n** | **Max** |
| 2.0 mg/kg | Day 1 | 2 | 1.1000 | 1.4142 | 0.100 | 1.1000 | 2.100 | | | | | | |
| | Day 8 | 2 | 1.3500 | 1.4849 | 0.300 | 1.3500 | 2.400 | 2 | 0.2500 | 0.07071 | 0.200 | 0.2500 | 0.300 |
| | Day 14 | 1 | 1.0000 | NA | 1.000 | 1.0000 | 1.000 | 1 | 0.9000 | NA | 0.900 | 0.9000 | 0.900 |
| | | | | | | | | | | | | | |
| 4.0 mg/kg | Day 1 | 2 | 0.7500 | 0.6364 | 0.300 | 0.7500 | 1.200 | | | | | | |
| | Day 8 | 2 | 1.5500 | 1.7677 | 0.300 | 1.5500 | 2.800 | 2 | 0.8000 | 1.13137 | 0.000 | 0.8000 | 1.600 |
| | Day 14 | 2 | 1.4500 | 1.6263 | 0.300 | 1.4500 | 2.600 | 2 | 0.7000 | 0.98995 | 0.000 | 0.7000 | 1.400 |
| | | | | | | | | | | | | | |
| 8.0 mg/kg | Day 1 | 2 | 1.4000 | 0.4242 | 1.100 | 1.4000 | 1.700 | | | | | | |
| | Day 8 | 2 | 0.8000 | 0.4242 | 0.500 | 0.8000 | 1.100 | 2 | -0.6000 | 0.00000 | -0.600 | -0.6000 | -0.600 |
| | Dav 14 | 2 | 1.7500 | 1.2020 | 0.900 | 1.7500 | 2.600 | 2 | 0.3500 | 1.62635 | -0.800 | 0.3500 | 1.500 |
| Day 1 and Day 8 are pre-dose levels | | | | | | | | | | | | | |

**Table 3D**

| **Summary Statistics of Observed Values and Change from Baseline Values - Interleukin 6 (pg/mL)** | | | | | | | | | | | | | |
|---|---|---|---|---|---|---|---|---|---|---|---|---|---|
| | **Observed Values** | | | | | | | **Change from Baseline** | | | | | |
| **Treatment** | **Day** | **N** | **Mean** | **SD** | **Min** | **Median** | **Max** | **N** | **Mean** | **SD** | **Min** | **Media n** | **Max** |
| 2.0 mg/kg | Day 1 | 2 | 177.50 0 | 249.184 | 1.300 | 177.50 | 353.70 0 | | | | | | |
| | Day 8 | 2 | 190.90 0 | 264.033 | 4.200 | 190.90 | 377.60 0 | 2 | 13.4000 | 14.8492 4 | 2.900 | 13.400 0 | 23.900 |
| | Day 14 | 1 | 82.400 0 | NA | 82.400 | 82.400 0 | 82.400 | 1 | 81.1000 | NA | 81.100 | 81.100 0 | 81.100 |
| | | | | | | | | | | | | | |
| 4.0 mg/kg | Day 1 | 2 | 13.650 0 | 12.9400 | 4.500 | 13.650 0 | 22.800 | | | | | | |
| | Day 8 | 2 | 111.65 0 | 153.937 | 2.800 | 111.65 | 220.50 0 | 2 | 98.0000 | 140.997 | -1.700 | 98.000 0 | 197.70 0 |
| | Day 14 | 2 | 97.550 0 | 134.421 | 2.500 | 97.550 0 | 192.60 0 | 2 | 83.9000 | 121.480 95 | -2.000 | 83.900 0 | 169.80 0 |
| | | | | | | | | | | | | | |
| 8.0 mg/kg | Day 1 | 2 | 104.75 0 | 49.851 | 69.500 | 104.75 0 | 140.00 0 | | | | | | |
| | Day 8 | 2 | 34.150 0 | 17.3241 | 21.900 | 34.150 0 | 46.400 | 2 | -70.6000 | 32.526 | -93.600 | -70.60 | -47.600 |
| | Day 14 | 2 | 182.10 0 | 177.05956.900 | | 182.10 0 | 307.30 0 | 2 | 77.3500 | 226.910 | -83.100 | 77.350 0 | 237.80 0 |
| Day 1 and Day 8 are pre-dose levels | | | | | | | | | | | | | |

*Complement Activation*: In order to determine the effect of TIMP-GLIA administration on complement activation, blood samples were collected from patients in Part B of the study at: pre-dose, 15 and 30 minutes into the infusion and at 24 hours after infusion. The levels of complement C3a, C5a and SC5B-9 were measured in samples of each subject by ELISA. Tables 4A-C show results by complement marker, dose group and subject.

**Table 4A**

| **C3a Results (ng/mL)** | | | | | | |
|---|---|---|---|---|---|---|
| | **2 mg/kg** | | **4 mg/kg** | | **8 mg/kg** | |
| **Subject** | **001-002-008** | **001-006-008** | **001-003-005** | **001-006-009** | **001-003-006** | **001-006-010** |
| **Time Point** | | | | | | |
| Day 1 - Pre | 15.8 | 48.7 | 16.6 | 15.7 | 6.6 | 30.7 |
| Day 1 15 Min | 92.2 | 63.1 | 225.3 | 76.8 | 43.0 | 86.8 |
| Day 1 30 Min | 143.3 | 96.2 | 168.7 | 151.6 | 47.7 | 120.9 |
| Day 2 | 17.8 | 33.6 | 18.2 | 15.9 | 14.6 | 9.3 |
| Day 8 B - Pre | 13.7 | 37.7 | 15.1 | 15.5 | 7.1 | 25.1 |
| Day 8 15 Min | 394.0 | 401.7 | 546.2 | 162.7 | 59.3 | 688.9 |
| Day 8 30 Min | 597.5 | 439.9 | 421.7 | 174.1 | 48.4 | 569.9 |
| Day 9 | 8.5 | 37.5 | 17.1 | 15.4 | 8.3 | 11.0 |

**Table 4B**

| **C5a Results (ng/mL)** | | | | | | |
|---|---|---|---|---|---|---|
| | **2 mg/kg** | | **4 mg/kg** | | **8 mg/kg** | |
| **Subject** | **001-002-008** | **001-006-008** | **001-003-005** | **001-006-009** | **001-003-006** | **001-006-010** |
| **Time Point** | | | | | | |
| Day 1 - Pre | 9.2 | 11.3 | 8.7 | 6.5 | 3.2 | 6.1 |
| Day 1 15 Min | 8.7 | 12.4 | 10.4 | 6.6 | 3.8 | 5.9 |
| Day 1 30 Min | 9.5 | 13.0 | 9.0 | 8.6 | 4.6 | 8.4 |
| Day 2 | 10.5 | 11.1 | 9.0 | 7.1 | 4.2 | 6.6 |
| Day 8 B - Pre | 9.0 | 13.7 | 8.3 | 5.5 | 4.2 | 7.4 |
| Day 8 15 Min | 12.4 | 14.1 | 10.1 | 6.6 | 4.5 | 8.9 |
| Day 8 30 Min | 12.5 | 12.6 | 10.9 | 6.7 | 3.8 | 8.8 |
| Day 9 | 10.3 | 12.0 | 11.1 | 7.1 | 3.3 | 8.0 |

**Table 4C**

| **SC5B-9 Results (ng/mL)** | | | | | | |
|---|---|---|---|---|---|---|
| | **2 mg/kg** | | **4 mg/kg** | | **8 mg/kg** | |
| **Subject** | **001-002-008** | **001-006-008** | **001-003-005** | **001-006-009** | **001-003-006** | **001-006-010** |
| **Time Point** | | | | | | |
| Day 1 - Pre | 97 | 122 | 119 | 72 | 136 | 88 |
| Day 1 15 Min | 121 | 216 | 452 | 131 | 243 | 242 |
| Day 1 30 Min | 274 | 303 | 455 | 350 | 298 | 512 |
| Day 2 | 113 | 158 | 81 | 85 | 154 | 74 |
| Day 8 B - Pre | 129 | 132 | 176 | 91 | 136 | 92 |
| Day 8 15 Min | 540 | 549 | 816 | 215 | 220 | 1333 |
| Day 8 30 Min | 990 | 978 | 1275 | 578 | 226 | 1849 |
| Day 9 | 102 | 86 | 106 | 101 | 151 | 100 |

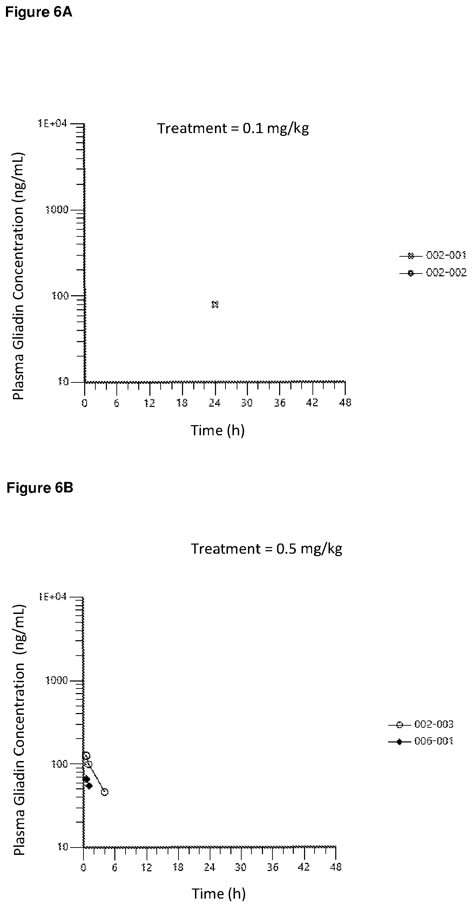

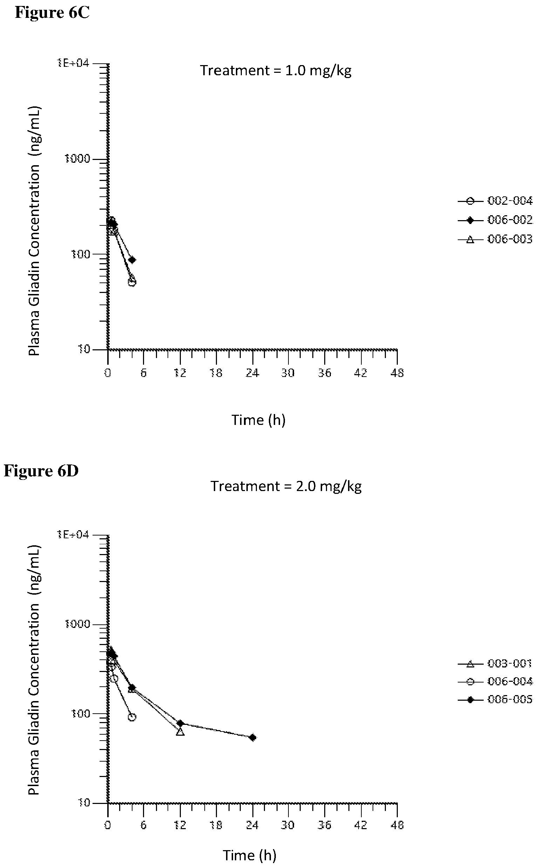

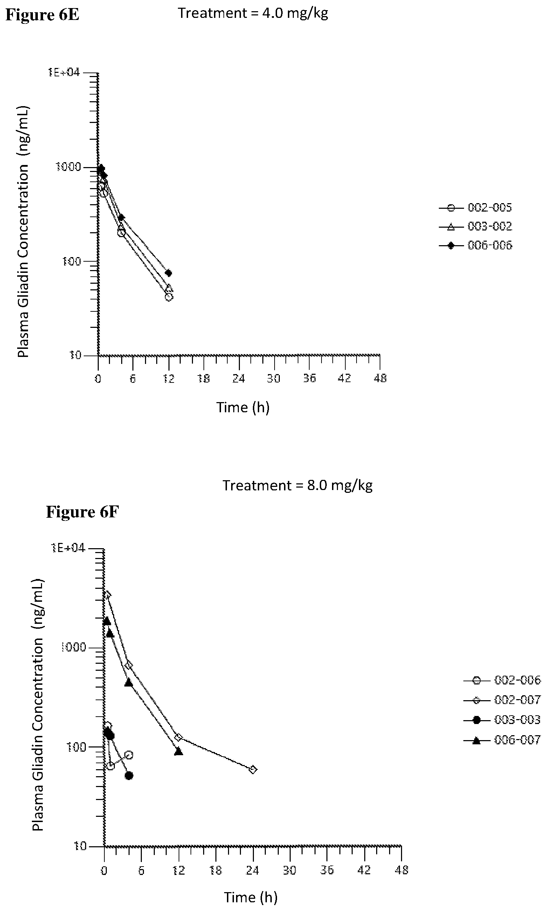

*TIMP-GLIA PK*: As noted above, plasma samples for PK were obtained at the following time points: PK pre-dose, 30 minutes, 35 minutes, end of infusion, 1, 4, 12, 24, 48, and 144 hours after each dose, for subjects in both Parts A and B. Individual subject plasma TIMP-GLIA concentrations over time (actual time elapsed from dosing) will be used to derive PK parameters using non-compartmental analysis: maximal observed concentration (Cₘₐₓ), last measurable concentration (Cₗₐₛₜ), time of maximal observed concentration (Tₘₐₓ), and area under curve from time zero and extrapolated to infinity (AUC_{inf}) and area under the concentration-time curve from time zero to time of last measurable concentration (AUCₗₐₛₜ). Individual subject plasma TIMP-GLIA concentrations, as measured by levels of plasma gliadin, over time and plotted by dose, are shown in Figures 6 A-F.

The data from the Phase 1 first-in-human multi-center clinical trial demonstrate that TIMP-GLIA administered intravenously in doses ranging from 0.1 to 8 mg/kg were generally safe and well-tolerated. The most common AEs reported were flushing, headache, back pain, fatigue, abdominal pain (n=2, 9%), and diarrhea. All AEs were mild to moderate. There was no evidence of immune activation as demonstrated by no increases in serum cytokines and chemokines, nor increases in gliadin-specific T cell proliferation and cytokine secretion in PBMCs.

Two of four subjects receiving a single dose of 8 mg/kg TIMP-GLIA experienced mild to moderate infusion reactions. No infusion reactions were observed in subjects receiving repeat doses of TIMP-GLIA (Part B). However, transient increases in complement levels (C3a and SC5b-9) were observed at 15 and 30 minutes into the infusion, but returned to baseline by 24 hours post-dose. None of the subjects had increases in serum cytokines and chemokines, gliadin-specific T cell proliferation or cytokine secretion, C1q binding, or DGP-IgG levels, suggesting that the reactions are not IgE-mediated but the result of complement activation-related pseudo-allergy (CARPA). Investigation into the mechanism behind complement increases is ongoing. The PK of TIMP-GLIA, as measured by plasma gliadin levels, appears to be dose-proportional and is back to baseline levels within 24 hours of administration.

Numerous modifications and variations in the disclosure as set forth in the above illustrative examples are expected to occur to those skilled in the art. Consequently only such limitations as appear in the appended claims should be placed on the disclosure.

### References

Anderson RP, van Heel DA, Tye-Din JA, et al. T cells in peripheral blood after gluten challenge in coeliac disease. Gut. 2005;54:1217-23.
Anderson RP, Degano P, Godkin AJ, et al. In vivo antigen challenge in celiac disease identifies a single transglutaminase-modified peptide as the dominant A-gliadin T-cell epitope. Nat Med. 2000; 6:337-342.
Baranwal AK, Singhi SC, Thapa BR, Kakkar N. Celiac Crisis. Indian J Pediatr. 2003;70:433-35.
Christophersen A, Risnes LF, Bergseng E, et al. Healthy HLA-DQ2.5+ subjects lack regulatory and memory t cells specific for immunodominant gluten epitopes of celiac disease. J Immunol. 2016;196(6):2819-26.
Farrell RJ, Kelly C. Celiac Sprue. N Engl J Med. 2002;346:180-8.
Fasano A, Catassi C. Celiac Disease. N Engl J Med. 2012;367:2419-26.
Fasano A, Catassi C. Current Approaches to Diagnosis and Treatment of Celiac Disease: An Evolving Spectrum. Gastroenterol. 2001;120:636-51.
Getts DR, Shea LD, Miller SD, King NJC. Harnessing nanoparticles for immune modulation. Trends Immunol. 2015;36(7):419-27.
Green PHR, Cellier C. Celiac Disease. N Engl J Med. 2007;357:1731-43.
Han A, Newell EW, Glanville J, et al. Dietary gluten triggers concomitant activation of CD4+ and CD8+ αβ T cells and γδ T cells in celiac disease. PNAS. 2013; 110(32):13073-8.
Kaukinen K, Lindfors K, Mäki M. Advances in the treatment of coeliac disease: an immunopathogenic perspective. Nat Rev Gastroenterol Hepatol. 2014;11:36-44.
Laurin P, Wolving M, Fälth-Magnusson K. J Ped Gastroenterol Nutr. 2002; 34:26-30
Leffler DA, Schuppan D, Pallav K, et al. Kinetics of the histological, serological and symptomatic responses to gluten challenge in adults with coeliac disease. Gut. 2013;62(7):996-1004.
Leffler DA, Acaster S, Gallop K, et al. A novel patient-derived conceptual model of the impact of celiac disease in adults: implications for patient-reported outcome and health-related quality-of-life instrument development. Value in Health. 2017;20:637-43.
Le Tourneau C, Lee JJ, Siu LL. Dose escalation methods in phase 1 cancer trials. J Natl Cancer Inst. 2009;101:708-20.
Makadia HK, Siegel SJ. Poly lactic-co-glycolic acid (PLGA) as biodegradable controlled drug delivery carrier. Polymers (Basel). 2011;3(3): 1377-97.
Mazzarella G, Stefanile R, Camarca A, Giliberti P, Cosentini E, Marano C et al. Gliadin activates HLA class I-restricted CD8+ T cells in celiac disease intestinal mucosa and induces the enterocyte apoptosis. Gastroenerol 2008;134:1017-27.
Rubio-Tapia A, Hill ID, Kelly CP et al. ACG Clinical Guidelines: Diagnosis and Management of Celiac Disease. Am J Gastroenterol. 2013;108:656-76. Rubinstein LV, Simon RM. Phase 1 Clinical Trial Design. In Handbook of Anticancer Drug Development. Budman DR, Calvert AH, Rowinsky EK (eds). Lippincott Williams & Wilkens, Baltimore, MD, 2003. pp. 297-308.
Steinman L. Inverse vaccination, the opposite of Jenner's concept, for therapy of autoimmunity. J Intern Med. 2010; 267: 441-51.
Suntharalingam G, Perry MR, Ward S, et al. Cytokine storm in a phase 1 trial of the anti-CD28 monoclonal antibody TGN1412. N Engl J Med 2006;355(10):1018-28.
Tack GJ, Verbeek WHM, Schreurs MWJ, Mulder CJJ. The spectrum of celiac disease: epidemiology, clinical aspects and treatment. Nat Rev Gastroenterol Hepatol. 2010;7:204-13. TIMP-GLIA Investigator's Brochure version 1 2017.
Wang Y, Qu W, Choi S. FDA's Regulatory Science Program for Generic PLA/ PLGA-Based Drug Products. Center for Drug Evaluation and Research FDA. Posted: June 15, 2016.

The present disclosure will now be further defined in the following paragraphs:
1. A method of treating Celiac Disease in a subject comprising administering to the subject a tolerizing immune modifying particle (TIMP) encapsulating one or more gliadin antigenic epitopes (GLIA), wherein the particle is administered at a dose of 0.1 to 10 mg/kg.
2. A method for reducing sensitivity to gluten in a subject comprising administering to the subject a tolerizing immune modifying particle (TIMP) encapsulating one or more gliadin antigenic epitopes (GLIA), wherein the particle is administered at a dose of 0.1 to 10 mg/kg.
3. The method of paragraph 1 or 2 wherein the TIMP-GLIA is administered in a single dose or in multiple doses.
4. The method of any one of the preceding paragraphs wherein the TIMP-glia is administered intravenously, subcutaneously, intramuscularly, intraperitoneally or orally.
5. The method of any one of the preceding paragraphs wherein the TIMP-GLIA comprises Poly(lactic-co-glycolic acid) (PLGA).
6. The method of any one of the preceding paragraphs wherein the TIMP-GLIA is carboxy functionalized on the surface.
7. The method of any one of the preceding paragraphs wherein the TIMP-GLIA has a negative zeta potential.
8. The method of any one of the preceding paragraphs wherein the zeta potential is between -80 to -30 mV.
9. The method of any one of the preceding paragraphs wherein the zeta potential is between -60 and -35 mV.
10. The method of any one of the preceding paragraphs wherein the TIMP-GLIA comprises Poly(lactic-co-glycolic acid) (PLGA) with a copolymer ratio of about 50:50 of polylactic acid:polyglycolic acid.
11. The method of any one of the preceding paragraphs wherein the particle size is between 100 and 1500 nm.
12. The method of any one of the preceding paragraphs wherein the particle size is between 100 and 1000 nm.
13. The method of any one of the preceding paragraphs wherein the particle size is between 400 and 800 nm.
14. The method of any one of the preceding paragraphs wherein the one or more GLIA antigens are selected from the group consisting of Gliadin, Glutenin, Hordein, Secalin
15. The method of any one of the preceding paragraphs wherein the subject is on a gluten free diet.
16. The method of any one of the preceding paragraphs wherein the TIMP-GLIA is infused over 30 minutes, 1 hour, 2 hours, 3 hours or more.
17. The method of any one of the preceding paragraphs wherein the TIMP-GLIA is infused at escalating rates.
18. The method of paragraphs 17 wherein the administration reduces complement activation compared to non escalating dose administration.
19. The method of any one of the preceding paragraphs wherein the subject has a genetic profile of HLA-DQ2.5 (HLA-DQA1*0501/B1*0201) or HLA-DQ8.1 (DQA1*0301/B1*0302).
20. The method of any one of the preceding paragraphs wherein the subject has Refractory Celiac Disease.
21. The method of any one of the preceding paragraphs wherein the administration improves one or more signs or symptoms of Celiac Disease or gluten sensitivity.
22. The method of paragraph 21 wherein the one or more symptoms is selected from the group consisting of weight loss, fatigue, headache, iron deficiency, folic acid deficiency, vitamin D deficiency, vitamin B12 deficiency, intestinal mucosal damage, and low bone density.
23. The method of paragraph 21 wherein the one or more signs of Celiac disease is identified from the group consisting of villous atrophy, tetramer staining, ELISPOT, miRNA, Exosomes, DNA and RNA.
24. The method of any one of the preceding paragraphs wherein the TIMP-GLIA is administered once weekly, once every two weeks, once every three weeks, once every 4 weeks, once every two months, once every three months, once every 6 months or once per year.
25. The method of any one of the preceding paragraphs wherein the TIMP-GLIA further comprises a pharmaceutical acceptable carrier, diluent or excipient.
26. The method of any one of the preceding paragraphs wherein the administration results in apoptosis of macrophages or monocytes in the subject.
27. The method of any one of the preceding paragraphs wherein the administration induces immunologic anergy.
28. The method of any one of the preceding paragraphs wherein the TIMP-GLIA is administered in conjunction with a second agent.
29. The method of any one of the preceding paragraphs wherein the TIMP-GLIA is administered as a booster dose.
30. The method of paragraph 29 wherein the booster dose is administered when needed as determined by skin-prick test or peripheral blood mononuclear cell levels.

## Claims

1. A tolerizing immune modifying particle (TIMP) encapsulating one or more gliadin antigenic epitopes (GLIA) for use in treating Celiac Disease or reducing sensitivity to gluten in a subject, wherein the subject is on a gluten free diet and the TIMP-GLIA is administered at a dose of 0.1 to 10 mg/kg.

2. The TIMP-GLIA for use of claim 1, which is formulated for administration intravenously, subcutaneously, intramuscularly, intraperitoneally or orally in a single dose or in multiple doses.

3. The TIMP-GLIA for use of claim 1 or 2 comprising Poly(lactic-co-glycolic acid) (PLGA).

4. The TIMP-GLIA for use of any one of claims 1-3, wherein the TIMP-GLIA is carboxy functionalized on the surface.

5. The TIMP-GLIA for use of any one of the preceding claims having a negative zeta potential between -80 to -30 mV or between 60 and -35 mV.

6. The TIMP-GLIA for use of claim 3, wherein the PLGA is a copolymer that has a ratio of about 50:50 of polylactic acid:polyglycolic acid.

7. The TIMP-GLIA for use of any one of the preceding claims wherein the particle size is between 100 and 1500 nm, between 100 and 1000 nm, or between 400 and 800 nm.

8. The TIMP-GLIA for use of any one of the preceding claims wherein the antigens are selected from the group consisting of Gliadin, Glutenin, Hordein, and Secalin.

9. The TIMP-GLIA for use of any one of the preceding claims, which is infused over 30 minutes, 1 hour, 2 hours, 3 hours or more; optionally is infused at escalating rates.

10. The TIMP-GLIA for use of any one of the preceding claims wherein the subject has a genetic profile of HLA-DQ2.5 (HLA-DQA1*0501/B1*0201) or HLA-DQ8.1 (DQA1*0301/B1 *0302).

11. The TIMP-GLIA for use of any one of the preceding claims wherein the administration improves one or more signs or symptoms of Celiac Disease or gluten sensitivity,
wherein the symptoms comprise weight loss, fatigue, headache, iron deficiency, folic acid deficiency, vitamin D deficiency, vitamin B12 deficiency, intestinal mucosal damage, or low bone density;
wherein the signs comprise villous atrophy, tetramer staining, ELISPOT, miRNA, Exosomes, DNA and RNA.

12. The TIMP-GLIA for use of any one of the preceding claims wherein the administration results in apoptosis of macrophages or monocytes in the subject.

13. The TIMP-GLIA for use of any one of the preceding claims wherein the administration induces immunologic anergy.

14. The TIMP-GLIA for use of any one of the preceding claims wherein the TIMP-GLIA is administered in conjunction with a second agent.
